# EUROPEAN PATENT APPLICATION

(11) **EP 2 754 652 A1**
(43) Date of publication of application: **16.07.2014**
(21) Application number: 13719217.5
(22) Date of filing: 19.02.2013
(51) Int. Cl.: C07C 309/14, C07C 303/02

(54) **TAURINE PREPARATION METHOD**

(30) Priority: 25.10.2012 CN 201210416911
(71) Applicant: Qiangjiang Yongan Pharmaceutical Co., Ltd., Qianjiang, Hubei 433102 (CN)
(72) Inventor: CHEN, Yong, Qianjiang Hubei 433102 (CN)
(74) Representative: Manitz, Finsterwald & Partner GbR
(86) International application number: PCT/CN2013/071665
(87) International publication number: WO 2014/063456

(57) **Abstract**

The present disclosure provides a process for producing taurine, includes: adjusting a PH value of a sodium taurate solution by a S⁴⁺ compound; introducing ethylene oxide into the sodium taurate solution to produce sodium hydroxyethyl sulfonate; separating crude taurine before or after introducing the ethylene oxide to the solution; and adding ammonia to the sodium hydroxyethyl sulfonate reaction solution to be reacted with the reaction solution to reproduce sodium taurate. The process for producing taurine of the present disclosure makes use of the balances of the sodium bases in the system, recycles the mother liquor until the sodium taurate is reproduced out of the reactions in the mother liquor, and thus is capable of allowing taurine to be synthesized and extracted. The process for producing taurine provided in the present disclosure makes full use of the material, avoids using a large amount of dangerous chemical material such as liquid caustic soda and sulfuric acid used in the traditional production process. This avoids producing a large amount of sodium sulfate solid waste, solves the problem that in the traditional production process the mother liquor needs to be concentrated many times and the discharged mother liquor causes environmental pollution, reduces the amount of vapor required for the many times concentrations of the mother liquor, improves the yield rate of the product, and reduces the production cost.

## Description

### BACKGROUND

### 1. Technical Field

The present invention relates to technologies of organic chemical synthesis, and particularly, to a process for producing taurine.

### 2. Description of Related Art

Taurine is a type of non-protein amino acid which can be used as an antiphlogistic, antipyretic, analgesic, anticonvulsant, and antihypertensive drug. Taurine is beneficial to the improvement of brain development, nerve conduction, visual function, and calcium absorption of a baby. Additionally, taurine has beneficial effects on the cardiovascular system and thus can strengthen health and remove fatigue. Therefore, taurine is commonly used in the fields such as medical treatment and food health, etc.

Taurine can be produced by using bio-extraction and chemical synthesis. Producing taurine by bio-extraction is rarely used due to the material and cost required thereby. Nowadays, in industrial production, there are two processes for producing taurine: by esterification of ethanolamine and by ethylene oxide. Compared to the process for producing taurine by esterification of ethanolamine, producing taurine by ethylene oxide has advantages including low cost, good quality, little environmental pollution, and continualness, which makes the process for producing taurine by ethylene oxide have a competitive advantage.

Producing taurine by ethylene oxide is a newly developed process for producing taurine and the principle thereof is as follows:

NaOH+SO₂+CH₂CH₂O→HOCH₂CH₂SO₃Na+NH₃→H₂NCH₂CH₂SO₃Na+H₂SO ₄→H₂ NCH₂CH₂SO₃H+Na₂SO₄

However, there are also some disadvantages of the process for producing taurine by ethylene oxide. Firstly, the material such as liquid caustic soda and sulfuric acid used in the process are dangerous chemical material which not only corrode pipeline and facilitate easily but also cause accidents easily during manual operations to further cause great economic loss. Secondly, during the process, a large amount of sodium sulfate solid waste is produced; as the sodium sulfate solid waste accumulates after a long time, some taurine is taken away, which causes loss of material; additionally, since the sodium sulfate exits in the mother liquors throughout, the sodium sulfate may block the cooler, heater, reactor, and high pressure pipeline easily in high temperature high pressure synthetic conditions, which prevents the normal production of taurine; at the same time, the large amount of sodium sulfate solid waste may increase working strength and is difficult to be handled. Thirdly, in the later extraction process, a large amount of waste mother liquor may be produced after many times of extractions of taurine (H₂NCH₂CH₂SO₃H). Fourthly, some mother liquor is required to be discharged, which not only causes the waste of the material and low efficiency, but also causes environmental pollution; meanwhile, a lot of vapors are consumed during the concentrating process of the mother liquor, which results in high energy consumption. Therefore, it requires for an effective method to overcome the above shortcomings.

### SUMMARY

The main object of the present disclosure is to provide a process for producing taurine, which makes full use of sodium bases in the system, recycles the mother liquor without adding new material or reagent, requires less dangerous material, greatly simplifies the production process of taurine, improves the utilization rate of the material and yield rate of the product, and reduces the production cost.

The process for producing taurine of the present disclosure includes:
(1) adjusting a PH value of a sodium taurate solution by a S⁴⁺ compound;
(2) introducing ethylene oxide into the sodium taurate solution to produce sodium hydroxyethyl sulfonate;
(3) separating crude taurine before or after introducing the ethylene oxide to the solution; and
(4) adding ammonia to the sodium hydroxyethyl sulfonate reaction solution to be reacted with the reaction solution to reproduce sodium taurate.

Preferably, a concentration of the sodium taurate solution ranges from 45% to 48% by weight percentage before the PH value is adjusted in the step (1).

Preferably, the S⁴⁺ compound used for adjusting the PH value in the step of (1) is SO₂ or H₂SO₃.

Preferably, the PH value is adjusted to range from 4.5 to 9.0 by SO₂ in the step (1).

Preferably, the PH value is adjusted to range from 5.5 to 6.5 by SO₂ in the step (1).

Preferably, a concentration of the sodium hydroxyethyl sulfonate ranges from 48% to 55% by weight percentage in the step (2).

Preferably, in the step (2), after introducing ethylene oxide into the sodium taurate solution with the adjusted PH value at a temperature ranging from 70°C to 80°C, and the ratio of the amounts of sodium taurate to ethylene oxide in the solution ranges from 1:1 to 1:1.2.

Preferably, in the step (4), after adding ammonia into the sodium hydroxyethyl sulfonate reaction solution, the ammonia accounts for 23∼26% by volume percentage of the mixed solution.

Preferably, the process for producing taurine is used for producing RNHCH₂CH₂So₃H and R(R')NCH₂CH₂SO₃H.

The process for producing taurine of the present disclosure makes use of the balances of the sodium bases in the system, recycles the mother liquor until the sodium taurate is reproduced out of the reactions in the mother liquor, and thus is capable of allowing taurine to be synthesized and extracted. The process for producing taurine provided in the present disclosure makes full use of the material, avoids using a large amount of dangerous chemical material such as liquid caustic soda and sulfuric acid used in the traditional production process. This avoids producing a large amount of sodium sulfate solid waste, solves the problem that in the traditional production process the mother liquor needs to be concentrated many times and the discharged mother liquor causes environmental pollution, reduces the amount of vapor required for the many times concentrations of the mother liquor, improves the yield rate of the product, and reduces the production cost.

### DETAILED DESCRIPTION

The disclosure is illustrated by way of example and not by way of limitation in the figures of the accompanying drawings in which like references indicate similar elements. It should be noted that references to "an" or "one" embodiment is this disclosure are not necessarily to the same embodiment, and such references mean at least one.

The present disclosure provides a process for producing taurine, including the following steps: adjusting a PH value of a sodium taurate solution by a S⁴⁺ compound, crystallizing the solution to separate crude taurine from the solution; introducing ethylene oxide into a mother liquor that is left over after the crystallization to produce sodium hydroxyethyl sulfonate; and thereafter ammonia is added to reproduce taurine tauroncholate.

Additionally, taurine can also be produced by the following steps: adjusting the PH value of the sodium taurate solution by a S⁴⁺ compound; and continuously introducing ethylene oxide to the sodium taurate solution to produce a mixed solution of sodium hydroxyethyl sulfonate and taurine, separating crude taurine from the mixed solution, and thereafter ammonia is added to the mother liquor to react with the mother liquor to reproduce the sodium taurate.

The reacting principle is as follows:

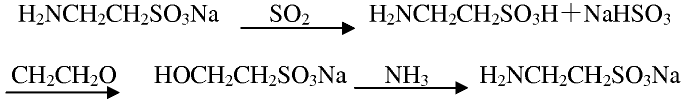

### EMBODIMENT 1

Getting 500 ml of a 46% sodium taurate solution in a reaction bottle; slowly introducing SO₂ into the sodium taurate solution under stirring until the PH value of the solution reaches 5.5; cooling the solution to the room temperature and filtering the solution to obtain crude taurine; placing the filtrate into the reaction bottle; slowly introducing ethylene oxide into the filtrate under stirring at 72°C (wherein the ratio of the amounts of sodium taurate to ethylene oxide in the solution is 1:1) until the reaction is terminated (which can be determined by iodine drops); adding 1015g of NH₃•H₂O into the reaction solution; heating the mixture of the reaction solution and NH₃•H₂O in a high pressure reactor until the temperature of the mixture reaches 250°C and the pressure of the mixture reaches 4.05MPA; keeping the reaction for an hour; cooling the mixture and moving the mixture out of the reactor and evaporating the mixture to get rid of ammonia to obtain the sodium taurate solution. The content of sodium taurate is measured by HPLC, the contents of SO₃²⁻ and sodium hydroxyethyl sulfonate are measured by chromatography of ions, and the result is shown as follows by weight percentage:
crude taurine: amount: 243g;
the content of taurine: 86.42%; and
the content of SO₃²⁻ : 1.98%; and
sodium taurate solution: amount: 738g;
the content of sodium taurate (corresponding to taurine): 39.16%; and
the content of sodium hydroxyethyl sulfonate: 48.5%.

### EMBODIMENT 2

Getting 500 ml of a 46% sodium taurate solution in a reaction bottle; slowly introducing SO₂ into the sodium taurate solution under stirring until the PH value of the solution reaches 5.5; slowly introducing ethylene oxide into the solution at 75°C (wherein the ratio of the amounts of sodium taurate to ethylene oxide in the solution is 1:1.1) until the reaction is terminated (which can be determined by iodine drops); cooling the solution to the room temperature to obtain crude taurine; adding 1005g of NH₃•H₂O to the reaction solution, heating the mixture of the reaction solution and NH₃•H₂O in a high pressure reactor until the temperature reaches 253°C and the pressure reaches 4.1MPa; keeping the reaction for an hour; cooling the mixture and moving the mixture out of the reactor and evaporating the mixture to get rid of ammonia to obtain sodium taurate solution. The result is shown as follows by weight percentage:
crude taurine: amount: 242g; and
the content of taurine: 86.78%;
the content of SO₃²⁻ : 1.9%; and
sodium taurate solution: amount: 616g;
the content of sodium taurate (corresponding to taurine): 39.15%; and
the content of sodium hydroxyethyl sulfonate: 49.62%.

### EMBODIMENT 3

Getting 500 ml of a 46% sodium taurate solution in a reaction bottle; slowly introducing SO₂ into the sodium taurate solution under stirring until the PH value of the solution reaches 6.0; cooling the solution to the room temperature and filtering the solution to obtain crude taurine; placing the filtrate into the reaction bottle and slowly introducing ethylene oxide into the filtrate under stirring at 78°C (wherein the ratio of the amounts of sodium taurate to ethylene oxide in the solution is 1:1) until the reaction is terminated (which can be determined by iodine drops); adding 1010g of NH₃•H₂O to the reaction solution; heating the mixture of the reaction solution and NH₃•H₂O in a high pressure reactor until the temperature reaches 255°C and the pressure reaches 4.15MPa; keeping the reaction for 1.5 hour; cooling the mixture and moving the mixture out of the reactor and evaporating the mixture to get rid of ammonia to obtain sodium taurate solution. The result is shown as follows by weight percentage:
crude taurine: amount: 249g;
the content of taurine: 86.34% ; and
the content of SO₃²⁻: 4.31%; and
sodium taurate solution: amount: 720g;
the content of sodium taurate (corresponding to taurine): 39.16%; and
the content of sodium hydroxyethyl sulfonate: 50.15%.

### EMBODIMENT 4

Getting 500 ml of a 46% sodium taurate solution in a reaction bottle; slowly introducing SO₂ into the sodium taurate solution under stirring until the PH value of the solution reaches 6.0; slowly introducing ethylene oxide into the solution at 80°C (wherein the ratio of the amounts of sodium taurate to ethylene oxide in the solution is 1:1.2) until the reaction is terminated (which can be determined by iodine drops); cooling the solution to the room temperature and filtering the solution to obtain crude taurine; adding 1010g of NH₃•H₂O to the reaction solution; heating the mixture of the reaction solution and NH₃•H₂O in a high pressure reactor until the temperature reaches 258°C and the pressure reaches 4.2MPa; keeping the reaction for 1.5 hour; and cooling the mixture and moving the mixture out of the reactor and evaporating the mixture to get rid of ammonia to obtain sodium taurate solution. The result is as follows by weight percentage:
crude taurine: amount: 246g;
the content of taurine: 85.36%;
the content of SO₃²⁻ : 3.76%;
sodium taurate solution: amount: 607g;
the content of sodium taurate (corresponding to taurine): 39.13%; and
the content of sodium hydroxyethyl sulfonate: 52.86%.

Even though information and the advantages of the present embodiments have been set forth in the foregoing description, together with details of the mechanisms and functions of the present embodiments, the disclosure is illustrative only; and that changes may be made in detail, especially in matters of shape, size, and arrangement of parts within the principles of the present embodiments to the full extend indicated by the broad general meaning of the terms in which the appended claims are expressed.

## Claims

1. A process for producing taurine, comprising:
(1) adjusting a PH value of a sodium taurate solution by a S⁴⁺ compound;
(2) introducing ethylene oxide into the sodium taurate solution to produce sodium hydroxyethyl sulfonate;
(3) separating crude taurine before or after introducing the ethylene oxide to the solution; and
(4) adding ammonia to the sodium hydroxyethyl sulfonate reaction solution to be reacted with the reaction solution to reproduce sodium taurate.

2. The process for producing taurine as claimed in claim 1, wherein a concentration of the sodium taurate solution ranges from 45% to 48% by weight percentage before the PH value is adjusted in the step (1).

3. The process for producing taurine as claimed in claim 1, wherein the S⁴⁺ compound used for adjusting the PH value in the step of (1) is SO₂ or H₂SO₃.

4. The process for producing taurine as claimed in claim 1, wherein the PH value is adjusted to range from 4.5 to 9.0 by SO₂ in the step (1).

5. The process for producing taurine as claimed in claim 4, wherein the PH value is adjusted to range from 5.5 to 6.5 by SO₂ in the step (1).

6. The process for producing taurine as claimed in claim 5, wherein a concentration of the sodium hydroxyethyl sulfonate ranges from 48% to 55% by weight percentage in the step (2).

7. The process for producing taurine as claimed in claim 1, wherein in the step (2), after introducing ethylene oxide into the sodium taurate solution with the adjusted PH value at a temperature ranging from 70°C to 80°C, and the ratio of the amounts of sodium taurate to ethylene oxide in the solution ranges from 1:1 to 1:1.2.

8. The process for producing taurine as claimed in claim 1, wherein in the step (4), after adding ammonia into the sodium hydroxyethyl sulfonate reaction solution, the ammonia accounts for 23∼26% by volume percentage of the mixed solution.

9. The process for producing taurine as claimed in any one from claim 1 to claim 8, wherein the process for producing taurine is used for producing RNHCH₂CH₂SO₃H and R(R')NCH₂CH₂SO₃H.
